Europäisches Patentamt

European Patent Office

Office européen des brevets

(19)

(11) Veröffentlichungsnummer : **0 143 472**
**B1**

(12) **EUROPÄISCHE PATENTSCHRIFT**

(45) Veröffentlichungstag der Patentschrift :
**05.04.89**

(21) Anmeldenummer : **84201157.9**

(22) Anmeldetag : **10.08.84**

(51) Int. Cl.⁴ : **C 07 C 37/74, C 07 C 39/04,
C 07 C 37/84, C 07 C 39/07**

(54) **Verfahren zur Aufarbeitung von Rohphenol aus der Kohleverflüssigung.**

(30) Priorität : **25.11.83 DE 3342665**

(43) Veröffentlichungstag der Anmeldung :
**05.06.85 Patentblatt 85/23** ⸱

(45) Bekanntmachung des Hinweises auf die Patenterteilung : **05.04.89 Patentblatt 89/14** ⸱

(84) Benannte Vertragsstaaten :
**DE FR GB IT NL**

(56) Entgegenhaltungen :
**GB-A- 920 864
GB-A- 1 479 124
US-A- 2 301 709
US-A- 2 971 893
CHEMICAL ABSTRACTS, Band 85, Nr. 11, 13. September 1976, Seite 522, Nr. 77865y, Columbus, Ohio, US;
& DD - A - 114 396 (W. KIESSLING et al.) 05.08.1975**

(73) Patentinhaber : **RÜTGERSWERKE AKTIENGESELLS-CHAFT
Mainzer Landstrasse 217
D-6000 Frankfurt am Main 1 (DE)**

(72) Erfinder : **Spengler, Hans, Dr.
Windmühlenweg 3
D-4716 Olfen (DE)**
Erfinder : **Stolzenberg, Konrad, Dr.
Insterburger Weg 5
D-4355 Waltrop (DE)**

EP 0 143 472 B1

**Beschreibung**

Die Erfindung betrifft ein Verfahren zur Aufarbeitung von Rohphenolfraktionen, die aus der Kohleverflüssigung stammen.

Zur Gewinnung von Phenol aus der Aufarbeitung fossiler Rohstoffe, z. B. der Steinkohle, wurden zahlreiche Verfahren entwickelt (vergl. H.-G. Franck, G. Collin ; Steinkohlenteer, Springer-Verlag, Berlin Heidelberg New York 1968, S. 74-83). Diese Verfahren sind alle auf die Phenolgewinnung aus Steinkohlenteer zugeschnitten und lassen sich nicht ohne weiteres auf entsprechende Fraktionen aus der Kohleverflüssigung übertragen. So können diese Fraktionen nicht mit dem Natronlauge-Extraktions-Verfahren aufgearbeitet werden, da sich dabei schwer zu brechende Emulsionen bilden, die Füllkörperkolonnen verschmutzen und bei der Begasung mit Kohlendioxid eine störende Schwarzfärbung der Sodalauge auftritt.

Infolgedessen werden die Phenole aus den genannten Fraktionen mit Hilfe des Phenoraffin-Verfahrens abgetrennt. Dabei werden die Phenole mit einer wäßrigen Lösung von Natriumphenolat aus der Ölfraktion extrahiert und aus dieser Lösung wiederum mit einem Lösungsmittel, meist Isopropyläther, extrahiert. Die dabei erhaltenen Rohphenole aber enthalten, im Gegensatz zu Rohphenolen, die aus Steinkohlenteer nach dem Phenoraffin-Verfahren gewonnen werden, größere Mengen an hochsiedenden Verbindungen, an organischen Basen, Schwefelverbindungen, Neutralölen und nicht identifizierten Verunreinigungen.

Diese störenden Begleiter des Phenolgemisches bedingen, daß sich bislang die Hauptkomponenten des Rohphenols nämlich Phenol, o-Kresol und m-/p-Kresol mit technischen Verfahren nicht in den gewünschten Reinheiten abtrennen lassen.

Aus US-A 2 301 709 ist ein Verfahren bekannt, mit dem Rohphenol aus Waschwasser verschiedener Verkokungs- und Kohlehydrierverfahren durch Behandlung mit Säure und Destillation und anschließender Behandlung mit Lauge und Destillation gereinigt wird. Über den Reinheitsgrad ist nichts ausgesagt. Es muß aber davon ausgegangen werden, daß sich mit diesem Verfahren keine Neutralöle aus Rohphenol abtrennen lassen.

Es ist daher die Aufgabe der Erfindung, ein großtechnisch durchführbares Verfahren zur Aufarbeitung von Rohphenolen zu finden, die aus der Kohleverflüssigung stammen, bei dem die Hauptkomponenten Phenol, o-Kresol und m-/p-Kresol in hoher Ausbeute und Reinheit gewonnen werden.

Die Lösung der Aufgabe erfolgt durch ein Aufarbeitungsverfahren gemäß der Ansprüche 1 - 5.

Es wurde gefunden, daß es durch diese umständlich anmutende Kombination der an sich bekannten Verfahrensschritte in der vorgeschriebenen Reihenfolge : Destillation des Rohphenols, Behandlung der Phenolfraktion mit Säure, vorzugsweise verdünnter Schwefelsäure, Verdampfung unter Vakuum, gegebenenfalls Behandlung des Destillats mit Peroxid und erneuter fraktionierter Destillation sowie Kristallisation der Hauptkomponenten Phenol und o-Kresol aus den Destillatfraktionen gelingt, die störenden Verunreinigungen nahezu vollständig abzutrennen, so daß reine Endprodukte erhalten werden. Gleichzeitig zeigte sich, daß diese Hauptkomponenten in hoher Ausbeute gewonnen werden und daß dieses erfindungsgemäße Verfahren in einer technischen Anlage wirtschaftlich durchgeführt werden kann. Darüber hinaus zeigte sich, daß mit diesem Verfahren die Rohphenole kontinuierlich aufgearbeitet werden können. Die einzelnen Verfahrensschritte können wie folgt charakterisiert werden :

Destillation des Rohphenols

Bedingt durch die besondere Behandlung der Phenolfraktionen aus der Kohleverflüssigung ist das aufzuarbeitende Rohphenol oft kein Destillat sondern der Rückstand, der bei der destillativen Trennung des Extraktionsgemisches aus Extraktionsmittel und Phenolen anfällt. Die in diesem Rohphenol enthaltenen hochsiedenden Bestandteile müssen in einem ersten Verfahrensschritt abdestilliert werden. Es wird daher eine Phenolfraktion im Siedebereich von 180 °C bis 220 °C unter Vakuum abdestilliert. Dabei verbleiben hochsiedende Phenole sowie ein Anteil der enthaltenen Basen in Rückstand.

Behandlung der Phenolfraktion mit Säure

Die Phenolfraktion aus dem Destillationsschritt enthält etwa 3 % Basen, die durch Behandeln mit Säure gebunden werden, z. B. was durch intensives Vermischen der Phenolfraktion mit verdünnter Schwefelsäure (10 bis 25 %) erfolgt.

Destillation

Zur Abtrennung der durch Schwefelsäurezugabe gebundenen Basen wird die Phenolfraktion unter Vakuum destilliert. Bei diesem Destillationsschritt fallen bis zu 10 % Rückstand an, bezogen auf die Menge der eingesetzten Phenolfraktion. Der Basengehalt des Destillats sinkt dadurch auf etwa 0,1 % und der Schwefelgehalt von 350 ppm auf ca. 60 ppm. Die gewählte Schrittfolge erscheint für den Fachman unzweckmäßig. Übliche Verfahrensweise wäre es, die Säurebehandlung der Phenolfraktion vor der Destillation des Rohphenols durchzuführen, um so eine zweite Destillation zu vermeiden. Dabei erfolgt jedoch eine relativ starke Verharzungsreaktion, die zu einer unverhältnismäßig hohen Menge (mehr als 20 %) an unerwünschten Rückstand führt. Es war für den Fachmann überraschend, daß die bei der üblichen Verfahrensweise gefundene starke Neigung zur Bildung von Rückständen durch die erfindungsgemäßen Verfahrensschritte wesentlich reduziert wird. Ebenfalls überraschend war es, daß es dabei auch gelingt, den Schwefelgehalt von 350 ppm and 60 ppm und weniger zu senken.

Wird dieser Verfahrensschritt als fraktionierte Destillation geführt, so werden aus dem vorgereinigten Phenolgemisch destillativ in an sich bekannter Weise Phenol, o-Kresol und m-/p-Kresol abgetrennt. Es ist daher ausreichend, bei Phenol und o-Kresol mit einem Rücklaufverhältnis von 5 : 1 und bei der Abtrennung von Xylenol mit einem Rücklaufverhältnis von 20 : 1 zu destillieren.

Überraschenderweise wird die fraktionierte Destillation wesentlich erleichtert gegenüber rohem Material, weil der Destillationseinsatz weitgehend basenfrei ist und sich keine störenden Azeotrope bilden können.

Mitunter enthalten die basenfreien Phenole aber noch Spuren weiterer Verunreinigungen, die zu Verfärbungen führen. Es wurde gefunden, daß diese Verunreinigungen durch zusätzliche Behandlung des nicht fraktionierten Destillats mit einem Oxidationsmittel so verändert werden können, daß sie dann in einer nachfolgenden Destillation nicht mehr als Begleiter der Endprodukte auftreten. Entsprechend wirksame Oxidationsmittel sind Ozon oder Peroxidvervindungen, insbesondere Wasserstoffperoxid, das in einer Menge von 0,2 - 3 % einer 30 %igen Lösung zum Destillat zugefügt wird. Die gewünschte Oxidationsreaktion erfolgt während der dann noch notwendig werdenden Destillation, in der das Phenolgemisch fraktioniert wird.

Kristallisation

Bedingt durch die besondere Herkunft der Rohphenole enthalten auch die aus der fraktionierten Destillation erhaltenen gereinigten Phenol- und o-Kresolfraktionen noch störende Verunreinigungen, insbesondere Neutralöle.

Die Abtrennung dieser schwer zu entfernenden Substanzen gelingt mittels fraktionierter Kristallisation.

Es wurde nun gefunden, daß eine optimale Abtrennung der Verunreinigungen erfolgt, wenn die Kristallisation in einer Vorrichtung erfolgt, in der die Kühlflächen geneigt zur Senkrechten angeordnet sind, und das flüssige Gemisch sich in Ruhe befindet oder maximal mit einer Geschwindigkeit bewegt wird, bei der gerade noch keine Kristallablösung von den Kühlflächen und kein Abbrechen der Kristallite aus der Kristallschicht stattfindet.

Dieses neue Verfahren nutzt die Tatsache aus, daß die dentritischen bzw. nadelförmigen Phenol- bzw. o-Kresolkristalle vorzugsweise in senkrechter Richtung zur Kühlfläche wachsen. Dieses Wachstum erfolgt wegen der relativ geringen Dichteunterschiede zwischen fester und flüssiger Phase nahezu unabhängig von der Erdanziehungskraft. An schräggestellten Kühlflächen wachsen die Kristalle also senkrecht zur Kühlfläche und daher, auf die Richtung der Erdanziehungskraft bezogen, schräg nach oben bzw. schräg nach unten. Diese Lage der Kristalle wirkt sich besonders günstig auf die Phasentrennung zwischen den Kristallen und der anhaftenden Restflüssigkeit aus. Dieses sogenannte « Tropföl » kann bei dem erfinderischen Verfahren in kürzerer Zeit und größeren absoluten Mengen aus der Kristallschicht auslaufen.

Das Verfahren kann in Kristallisationsapparaten mit schräg zur Senkrechten stehenden, parallel angeordneten Platten oder Rohren durchgeführt werden, die sowohl kühlbar als auch heizbar sein müssen und mit Flossen, Zähnen oder in Längsrichtung verlaufenden Rippen versehen sein können.

Es ist jedoch ebenfalls möglich, Kristallisationsapparate mit schräg zur Waagrechten eingebauten Rohren, die mit radial angeordneten Rippen oder Lamellen versehen sind, oder mit waagerecht angeordneten Rohren zu verwenden, die mit schräg gestellten Rippen oder Flügeln versehen sind.

Rippenrohre sind besonders vorteilhaft bei der Kristallisation aus ruhenden Flüssigkeitsgemischen, um den Wärmeübergang zu verbessern. Um eine gute Trennwirkung zwischen Tropföl und Kristallgut zu erzielen, ist bereits eine Neigung der wärmeübertragenden Fläche von 5° zur senkrechten Achse ausreichend. Neigungswinkel von über 70 Grad ördern zwar das Ablaufen der flüssigen Phase vom Einzelkristall, können aber die Ablaufgeschwindigkeit der flüssigen Phasen entlang der tiefer gelagerten Flächen reduzieren. Es wurde gefunden, daß der optimale Neigungswinkel zwischen 10 und 45 Grad liegt. Die fraktionierte Kristallisation wird in an sich bekannter Weise durchgeführt : Nach dem Abscheiden der Kristalle an den indirekt gekühlten Wänden der Kristallisationszone wird zunächst die nicht kristallisierte Flüssigkeit abgelassen. Anschließend wird durch programmgesteuerte Temperatureröhung die Kristallschicht abgeschmolzen, wobei das geschmolzene Kristallgut in Fraktionen entnommen werden kann.

Beim Abschmelzen der einzelnen Fraktionen werden aufgrund der Wärmezufuhr über die Wärmetauscherflächen die Haftkräfte der Kristallschicht aus diesen Flächen stark gemindert. Bei senkrechten Wärmetauscherflächen führt dies zu einem Abrutschen der gesamten Kristallmasse. Das hat Verstopfungen innerhalb des Kristallisators oder des Ablaufventils zur Folge. Die Gefahr des unerwünschten Abrutschens der Kristallschicht wird durch die Neigung der Wärmetauscherflächen ebenfalls gemindert. Einer Verstopfung des Ablaufbodens wird außerdem durch Beheizen dieses Teils vorgebeugt.

Da für die geforderten Trennaufgaben die einstufige Kristallisation nicht ausreichend ist, wird die fraktionierte Kristallisation mehrstufig ausgeführt, um eine höhere Reinheit zu erzielen.

Die mehrstufige fraktionierte Kristallisation kann sukzessive in ein- und derselben Anlage erfolgen, wobei die Restschmelzen bzw. -Lösungen und die einzelnen Fraktionen des abgeschmolzenen Kristalles aus den einzelnen Stufen in entsprechenden Gefäßen gespeichert werden. Sie kann aber auch in einer Reihe baugleicher, hintereinandergeschalteter Kristallisationsapparate durchgeführt werden.

Die fraktionierte Kristallisation wird in Zyklen durchgeführt, wobei jeder Zyklus aus mehreren Stufen bestehen kann. Die Restschmelze, sowie die einzelnen, durch partielles Abschmelzen gewonnenen

3

Fraktionen mit geringerer Reinheit werden jeweils dem Einsatzprodukt der Stufe des folgenden Zyklus zugeführt, die der jeweiligen Konzentration entspricht. Das Restkristall dient als Einsatzmaterial für die folgende Stufe desselben Zyklus. Zum Abschmelzen des Restkristalls kann überlicherweise die Schmelze der gleichen mittleren Konzentration aus dem vorangegangenen Zyklus verwendet werden.

Das erfindungsgemäße Verfahren wird durch das folgende Beispiel weiter erläutert. Alle Prozentangaben sind Angaben in Gewichtsprozenten.

Beispiel

Ein Rohphenol, erhalten nach dem Phenoraffin-Verfahren aus entsprechenden Fraktionen, die bei der Steinkohleverflüssigung anfallen, wird über Helm destilliert, wobei die Phenolfraktion als Kopfprodukt gewonnen wird (89,3 % des Rohphenols). Im Sumpf verbleiben 9,7 % Rückstand. Die Phenolfraktion wird mit 10 % ihres Gewichts 25 %iger Schwefelsäure versetzt und intensiv vermischt. Danach wird die Phenolphase unter Vakuum (150 mbar) fraktioniert destilliert. Bei diesem kombinierten Verfahrensschritt wird folgendes Ergebnis erzielt :

Einsatz :

| Gemisch aus Phenolfraktion und 10 % - 25 %iger Schwefelsäure | 2 796 g |
|---|---|
| Destillation | |
| wässriger Vorlauf | 295,7 g |
| Summe der entbasten Phenole | 2 321 g |
| | (0,092 % Basen) |
| Rückstand | 172 g |
| Verlust | 7,3 g |

Die Phenolprodukte sind wie folgt aufgegliedert :

| 57,8 % | Phenol-Fraktion |
|---|---|
| 17,1 % | o-Kresol-Fraktion |
| 32,8 % | m-/p-Kresol-Fraktion |
| 7,7 % | Dimethyl-, Trimethylphenol, und höher alkylierte Phenole |

Phenol- und o-Kresolfraktion enthalten noch Neutralöle. Zur weiteren Reinigung werden diese beiden Produkte getrennt jeweils einer fraktionierten Kristallisation unterworfen :

Die Schmelzen der Phenol- und der o-Kresolfraktion werden jeweils in ein quaderförmiges Kristallisiergefäß gefüllt, in dem sich parallel zueinander angeordnete Rohre befinden, die mit einem Kühl- bzw. Heizmedium durchströmt werden. Diese Rohre besitzen zwei gegebüberliegende, längs angeschweißte Rippen und sind so in dem Kasten installiert, das durch die Längsrippen der Rohre eine wärmeübertragende, große Fläche gebildet wird.

Die Rippen bilden einen Winkel von 10 Grad zur Senkrechten. Der Abstand zwischen zwei gegenüberliegenden Flächen beträgt 60 mm.

Nach Einfüllung der Phenol-(bzw. o-Kresol)schmelze in den Kristaller wird die Temperatur des Kühlwassers von 40 °C (30 °C) auf 10(5) °C während 90 min mit einem Gradienten von 0,156 °C/min erniedrigt. Nach Erreichen der Kühltemperatur wird das Bodenventil geöffnet, um den nicht kristallisierten Rückstand ablaufen zu lassen. Nach einer Haltezeit von 30 min bei 10(5) °C wird die Temperatur langsam mit einem Gradienten von 0,2 °C/min auf 37(28) °C erhöht. Bei 37(28) °C wird die Temperatur solange gehalten, bis die gewünschte Flüssigkeitsmenge abgelaufen ist. Die gereinigten Kristalle werden durch Temperaturerhöhung auf 60(50°) °C rasch abgeschmolzen. Zur Verkürzung der Abschmelzzeit wird die Schmelze über einen äußeren Kreislauf im Kristaller umgewälzt und dabei über einen Wärmetauscher ebenfalls erwärmt.

Bei diesem Verfahrensschritt werden 93 % Phenol (bezogen auf die eingesetzte Phenolfraktion) in 99,9 %iger Reinheit und 91 % o-Kresol (bezogen auf die eingesetzte o-Kresolfraktion) in 99,9 %iger Reinheit erhalten.

**Patentansprüche**

1. Verfahren zur Aufarbeitung von Rohphenol, das nach dem Phenoraffin-Verfahren aus phenolhaltigen Fraktionen aus der Kohleverflüssigung gewonnen wurde, dadurch gekennzeichnet, daß die Phenole von hochsiedenden Begleitern destillativ abgetrennt, mit verdünnter Säure versetzt und anschließend einer fraktionierten Destillation und die gewonnenen Phenol- und o-Kresol-Fraktionen einer Kristallisation unterworfen werden.

2. Verfahren nach Anspruch 1, dadurch gekennzeichnet, daß die mit verdünnter Säure versetzten Phenole unter Vakuum destilliert, mit einem Oxidationsmittel behandelt und danach einer fraktionierten Destillation und Kristallisation unterworfen werden.

3. Verfahren nach Anspruch 1 und 2, dadurch gekennzeichnet, daß die Aufarbeitung kontinuierlich erfolgt.

4. Verfahren nach Anspruch 1 bis 3, dadurch gekennzeichnet, daß die Kristallisation in einer Vorrichtung erfolgt, in der die Kühlflächen zur Senkrechten oder Waagerechten geneigt angeordnet sind, und daß sich das flüssige Gemisch in Ruhe befindet oder maximal mit einer Geschwindigkeit bewegt wird, bei der gerade noch kein Abbrechen der Kristallite stattfindet.

5. Verfahren nach Anspruch 1 bis 4, dadurch gekennzeichnet, daß die Kristallisation in einer Vorrichtung erfolgt, in der die Kühlflächen in einem Winkel von 10 bis 45° angeordnet sind.

## Claims

1. A process for the treatment of crude phenol which has been recovered in accordance with the phenoraffin process from phenol-containing fractions from the liquefaction of coal, characterized in that the phenols are separated by distillation from high-boiling accompanying substances, reacted with diluted acid and subsequently subjected to a fractionated distillation and the phenol and o-cresol fractions obtained are subjected to crystallization.

2. A process according to Claim 1, characterized in that the phenols reacted with diluted acid are distilled under vacuum, are treated with an oxidizing agent and are then subjected to a fractionated distillation and crystallization.

3. A process according to Claims 1 and 2, characterized in that the treatment takes place continuously.

4. A process according to Claims 1 to 3, characterized in that the crystallization takes place in an apparatus in which the cooling surfaces are arranged inclined with respect to the vertical or horizonal, and the liquid mixture is left at rest or at most is moved at a speed at which there is not yet any breaking of the crystallites.

5. A process according to Claims 1 to 4, characterized in that the crystallization takes place in an apparatus in which the cooling surfaces are arranged at an angle of from 10 to 45°.

## Revendications

1. Procédé de traitement de phénol brut obtenu par le procédé phénoraffin à partir de fractions contenant du phénol survenant lors de la liquéfaction du charbon, caractérisé par le fait que les phénols sont séparés par distillation des produits d'accompagnement à point d'ébullition élevé, additionnés d'acide dilué et ensuite soumis à une distillation fractionnée, les fractions de phénol et d'ocrésol ainsi obtenues étant soumises à une cristallisation.

2. Procédé selon la revendication 1, caractérisé par le fait que les phénols additionnés d'acide dilué sont distillés sous vide, traités avec un agent d'oxydation et ensuite soumis à une distillation et à une cristallisation fractionnées.

3. Procédé selon les revendications 1 et 2, caractérisé par le fait que le traitement est effectué en continu.

4. Procédé selon les revendications 1 à 3, caractérisé par le fait que la cristallisation a lieu dans une installation dans laquelle les surfaces de refroidissement sont disposées de manière inclinée par rapport à la verticale ou à l'horizontale et par le fait que le mélange liquide est au repos ou est au plus déplacé avec une vitesse pour laquelle il ne se produit pas encore de séparation par rupture des cristallites.

5. Procédé selon les revendications 1 à 4, caractérisé par le fait que la cristallisation a lieu dans une installation dans laquelle les surfaces de refroidissement sont disposées suivant un angle de 10 à 45°.